# EUROPEAN PATENT APPLICATION

(11) **EP 3 432 253 A1**
(43) Date of publication of application: **23.01.2019**
(21) Application number: 16894372.8
(22) Date of filing: 16.03.2016
(51) Int. Cl.: G06Q 50/22, G06Q 30/06

(54) **MEDICAL SYSTEM AND PROGRAM**

(71) Applicant: Reasonwhy Inc., Tokyo 105-0001 (JP)
(72) Inventor: SHIWAKU, Tetsuo, Tokyo 105-0001 (JP)
(74) Representative: Pfenning, Meinig & Partner mbB
(86) International application number: PCT/JP2016/058280
(87) International publication number: WO 2017/158754

(57) **Abstract**

[Problem to be Solved]

To provide a medical system in which a patient can select an appropriate physician for his or her disease and a physician can select an appropriate patient according to his or her technique.

[Solution]

A medical system 1 comprises: a patient disease condition acquisition unit 22a for acquiring patient disease condition information specifying disease condition of a patient; a data recording unit 23 for storing physician information relating to physicians; and a control unit 22 for matching a patient and a physician with each other on the basis of the patient disease condition information acquired by the patient disease condition acquisition unit 22a and the physician information stored in the data recording unit 23.

## Description

### [Technical Field]

The present invention relates to a medical system and a program.

### [Background Art]

A physician evaluation system for evaluating a physician by another physician, which can evaluate capability of the physician in each specialized field of the physician, for example, has been proposed (see Patent Literature 1, for example). According to such physician evaluation system, a medical facility such as a hospital and a clinic which employs a physician can recognize personality and capability of the physician on the basis of the evaluation of the physician, and thus can select a physician required for providing high-quality medical practice.

### [Prior Art Document]

### [Patent Document]

Patent Literature 1: Japanese Patent Laid-Open No. 2004-355613

### [Summary of the Invention]

### [Problems to be Solved by the Invention]

Here, not only the medical facility wants a physician with capability but also a patient wants an appropriate physician having capability relating to his or her disease. However, since the patient conventionally has no way to get in direct contact with such appropriate physician, the patient needs to search an appropriate physician through introduction by a general practitioner or a practitioner in town or the like, for example, which takes a long way and is difficult. The physician evaluation system according to Patent Literature 1 is used only for connecting the medical facility to the physician as described above and is not used for connecting the patient to the physician. Therefore, the patient could not have selected a physician appropriate for his or her disease, while the physician could not have selected an appropriate patient according to his or her technique.

The present invention was made in view of the above and has an object to provide a medical system in which a patient can select an appropriate physician for his or her disease and a physician can select an appropriate patient according to his or her technique.

### [Means for Solving the Problems]

In order to solve the above problem and achieve the above object, a medical system according to claim 1, comprising: patient disease condition acquisition unit for acquiring patient disease condition information specifying disease condition of a patient; physician information storage unit for storing physician information relating to a physician; and matching unit for matching the patient and the physician with each other on the basis of the patient disease condition information acquired by the patient disease condition acquisition unit and the physician information stored in the physician information storage unit.

The medical system according to claim 2 is the medical system according to claim 1, wherein the physician information storage unit comprises physician evaluation storage unit for storing physician evaluation information specifying evaluation specific to the physician; the matching unit comprises physician extraction unit for extracting a target physician who is a physician suitable for medical treatment of the patient on the basis of the patient disease condition information acquired by the patient disease condition acquisition unit and the physician evaluation information stored in the physician evaluation storage unit; and the medical system comprises: target physician information storage unit for storing information relating to the target physician extracted by the physician extraction unit; and output unit for outputting information relating to the target physician stored in the target physician information storage unit.

The medical system according to claim 3 is the medical system according to claim 2, wherein the patient disease condition information acquired by the patient disease condition acquisition unit is an ICD disease name, which is a disease name determined in accordance with the International Classification of Diseases; and the physician evaluation storage unit stores evaluation of the physician in relation to a disease corresponding to the ICD disease name as the physician evaluation information.

The medical system according to claim 4 is the medical system according to claim 2 or 3, the physician evaluation storage unit stores evaluation of the physician in relation to a treatment method corresponding to the disease condition of the patient as the physician evaluation information.

The medical system according to claim 5 is the medical system according to any one of claims 2 to 4, further comprising: patient attribute acquisition unit for acquiring patient attribute information specifying an attribute of the patient; physician attribute acquisition unit for acquiring physician attribute information specifying an attribute of the physician; and matching degree calculation unit for calculating a matching degree indicating a degree for the physician to be suitable for treating the patient on the basis of the patient attribute information acquired by the patient attribute acquisition unit and the physician attribute information acquired by the physician attribute acquisition unit, wherein the physician extraction unit extracts the target physician on the basis of the matching degree calculated by the matching degree calculation unit, the patient disease condition information acquired by the patient disease condition acquisition unit, and the physician evaluation information stored in the physician evaluation storage unit.

The medical system according to claim 6 is the medical system according to claim 5, wherein the attribute of the patient includes age, sex, a current location or a medical treatment fee of the patient.

The medical system according to claim 7 is the medical system according to any one of claims 2 to 6, wherein the evaluation specific to the physician is evaluation by physicians other than the physician concerned.

The medical system according to claim 8 is the medical system according to any one of claims 1 to 7, further comprising: symptom storage unit for storing, in association with each other, disease name information specifying a disease name and symptom information specifying a symptom involved in the disease, wherein the patient disease condition acquisition unit acquires a symptom involved in the disease of the patient; and the matching unit specifies the disease name information stored in the symptom storage unit on the basis of the symptom acquired by the patient disease condition acquisition unit and the symptom information stored in the symptom storage unit, and matches the patient and the physician with each other on the basis of the specified disease name information and the physician information stored in the physician information storage unit.

The medical system according to claim 9 is the medical system according to any one of claims 1 to 8, wherein a plurality of the patients makes inputs relating to their own disease conditions by input unit, the patient disease condition acquisition unit specifies the disease condition of each of the patients on the basis of input contents input by the patients; and the matching unit matches each of the patients and the physician with each other by: extracting input contents relating to medical treatment of the physician from the input contents of the plurality of patients on the basis of the physician information relating to the physician; and outputting the extracted input contents to the physician.

A medical program according to claim 10, the medical program which causes a computer to function as: patient disease condition acquisition unit for acquiring patient disease condition information specifying disease condition of a patient; physician information storage unit for storing physician information relating to a physician; and matching unit for matching the patient and the physician with each other on the basis of the patient disease condition information acquired by the patient disease condition acquisition unit and the physician information stored in the physician information storage unit.

### [Advantage of the Invention]

According to the medical system according to claim 1 or the medical program according to claim 10, since the patient and the physician are matched with each other on the basis of the disease condition information and the physician information, matching can be made on the basis of the disease condition of the patient and the information of the physician, and the patient can select a physician appropriate for his/her own disease or the physician can select an appropriate patient in accordance with his/her own techniques.

According to the medical system according to claim 2, since the information relating to a target physician is output on the basis of the patient disease condition information and the physician evaluation condition, a physician determined to be objectively excellent can be output with priority in selecting a target physician, and the patient can select a physician more appropriate for his/her own disease.

According to the medical system according to claim 3, since the evaluation of the physician in relation to a disease corresponding to the ICD disease name is stored, the evaluation of the physician relating to the internationally regulated reference can be stored, and a single integrated evaluation can be stored even for a disease having a plurality of disease names.

According to the medical system according to claim 4, since the evaluation of the physician in relation to a treatment method corresponding to the disease condition of the patient is stored, even if a plurality of different diseases can be treated by a single treatment method or the like, the evaluation of a physician can be appropriately stored.

According to the medical system according to claim 5, the matching degree indicating a degree for the physician to be suitable for medical treatment of the patient is calculated on the basis of the patient attribute information and the physician attribute information, and the target physician is extracted by considering this matching degree and thus, the target physician can be extracted on the basis of the information other than the patient's disease condition or the evaluation of the physician and the information specific to the patient and the physician, and the patient can select a physician more appropriate for a treatment the patient receives for the his/her own disease.

According to the medical system according to claim 6, since the attribute of the patient includes age, sex, a current location or a medical treatment fee of the patient, the target physician can be extracted on the basis of the specific information such as the age, sex, current location or treatment fee of the patient, and the patient can select a physician more appropriate for a medical treatment the patient receives for the his/her own disease.

According to the medical system according to claim 7, since the evaluation of the physician is determined on the basis of evaluation from another physician who has specialized knowledge, more appropriate evaluation can be stored.

According to the medical system according to claim 8, since the disease name of the patient can be specified on the basis of the symptom of the patient, and the physician corresponding to the specified disease name and the patient can be matched with each other, even if the patient does not grasp the disease name or cannot determine by himself/herself whether the patient is sick or not, the patient and the physician can be matched with each other appropriately.

According to the medical system according to claim 9, after the disease condition of the patient is specified on the basis of the input contents of the patient, the input contents are output to the physician determined to be appropriate on the basis of the disease condition and thus, the physician can contact the patient suitable for the specialty of the physician upon reception of the input contents, and the physician can select a patient appropriate for his/her own techniques easily.

### [Brief Description of the Drawings]

[Fig. 1] Fig. 1 is a block diagram exemplifying a medical system according to an embodiment of the present invention.
[Fig. 2] Fig. 2 is a view illustrating a configuration example of an ICD disease name DB.
[Fig. 3] Fig. 3 is a view illustrating a configuration example of a physician evaluation DB.
[Fig. 4] Fig. 4 is a view illustrating a configuration example of a physician attribute DB.
[Fig. 5] Fig. 5 is a view illustrating a configuration example of a target physician DB.
[Fig. 6] Fig. 6 is a flowchart of physician evaluation DB creation processing.
[Fig. 7] Fig. 7 is a flowchart of physician attribute DB creation processing.
[Fig. 8] Fig. 8 is a flowchart of physician evaluation processing.
[Fig. 9] Fig. 9 is a flowchart of target physician output processing.
[Fig. 10] Fig. 10 is a view illustrating a configuration example of physician search information.
[Fig. 11] Fig. 11 is a flowchart of target physician extraction processing.
[Fig. 12] Fig. 12 is a flowchart of sorting processing.
[Fig. 13] fig. 13 is a view conceptually illustrating a medical system according to a variation 1.
[Fig. 14] Fig. 14 is a view illustrating a configuration example of a coordinator DB according to a variation 2.
[Fig. 15] Fig. 15 is a view illustrating a configuration example of a symptom DB according to a variation 3.

### [Detailed Description of Embodiments]

Embodiments of a medical system and a program according to the present invention will be described below in detail with referring to the attached drawings. However, the present invention is not limited by these embodiments.

### (Configuration)

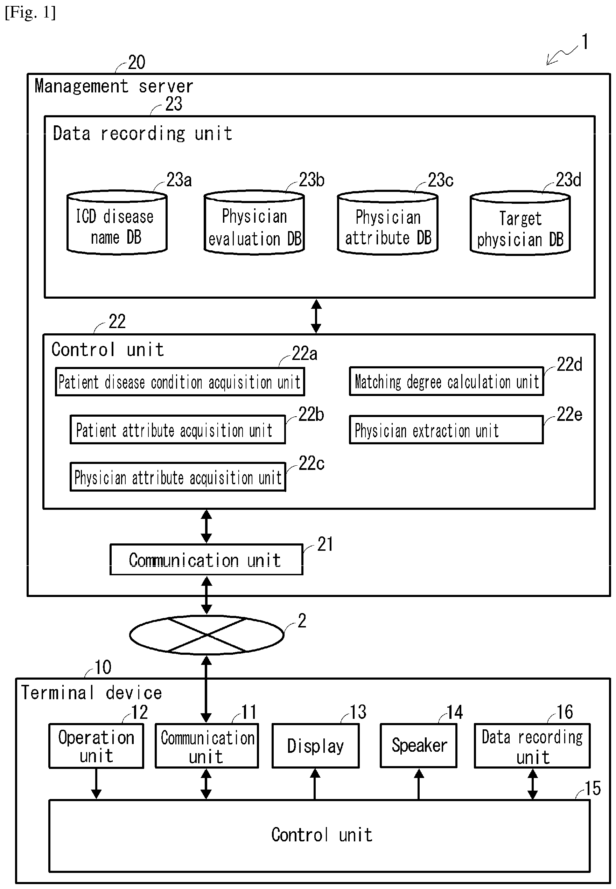

First, configuration of a medical system 1 according to an embodiment will be described. Fig. 1 is a block diagram exemplifying the medical system 1 according to this embodiment. Schematically, this medical system 1 is configured to comprise a terminal device 10 and a management server 20, and the terminal device 10 and the management server 20 are connected to be capable of communication with each other via a network 2. Here, only one unit of the terminal device 10 is illustrated in Fig. 1, but in actuality, the medical system 1 is configured to comprise a plurality of the terminal devices 10 and a common management server 20 capable of communication with the plurality of terminal devices 10. However, since the plurality of terminal devices 10 can be configured similarly to each other, the one unit of the terminal device 10 will be described below, while description for the other terminal devices 10 will be omitted.

### (Configuration - terminal device)

Subsequently, configuration of the terminal device 10 will be described. The terminal device 10 is a device which outputs patient disease condition information and patient attribute information to the management server 20 and executes various controls based on the information output from the management server 20. Here, the "patient disease condition information" is information for specifying disease condition of a patient. Here, the "disease condition" is a concept including vague "physical condition" of the patient, a "symptom" involved in a disease, a "general disease name (cancer, diabetes and the like) which is a generally known disease name, and an "ICD disease name (hypertensive disease, acute myocardial infarction and the like) which is a disease name defined in accordance with the International Classification of Diseases, although the "disease condition" in this embodiment is assumed to be an "ICD disease name". Moreover, the "patient attribute information" is information for specifying an attribute of the patient and is a concept including information for specifying an age of the patient, information specifying the sex of the patient, information specifying a location of the patient, and information relating to a budget which can be used for treatment, for example.

Moreover, this terminal device 10 is configured by using a known mobile terminal such as a mobile phone terminal including a smart phone (or may be configured by using a known personal computer, a tablet computer or the like) and comprises a communication unit 11, an operation unit 12, a display 13, a speaker 14, a control unit 15, and a data recording unit 16. Since the terminal device 10 can be configured by a known mobile terminal, for example, detailed description thereof will be omitted.

### (Configuration - terminal device - communication unit)

The communication unit 11 is communication unit for communicating with the management server 20 via the network 2. This communication unit 11 can be configured as known communication unit conducting communication using a mobile radio communication network, for example.

### (Configuration - terminal device- operation unit)

The operation unit 12 is operating unit for accepting an operation input from a user. This operation unit 12 can be configured as remote operating unit such as a touch panel and a remote controller or known operating unit such as a hard switch.

### (Configuration - terminal device - display)

The display 13 is display unit for displaying various images on the basis of the control of the control unit 15. As this display 13, a flat panel display such as a known liquid crystal display or an organic EL display can be used.

### (Configuration - terminal device - speaker)

The speaker 14 is output unit for outputting various sounds on the basis of the control of the control unit 15. A specific mode of the sound output from the speaker 14 is arbitrary, and synthesized sound generated as necessary or sound recorded in advance can be output.

### (Configuration - terminal device - control unit)

The control unit 15 is control unit for controlling the terminal device 10 and specifically, is a computer configured to comprise a CPU, various programs interpreted/executed on the CPU (including a basic control program such as OS and an application program activated on the OS and realizing a specific function), and an internal memory such as a RAM for storing the programs and various types of data. Particularly, a medical program according to the embodiment is installed in the terminal device 10 through an arbitrary recording medium or via the network 2 so as to configure each part of the control unit 15 by being (it is assumed that the same applies to a control unit 22 of the management server 20 described later).

### (Configuration - terminal device - data recording unit)

The data recording unit 16 is recording unit for recording a program and various types of data required for an operation of the terminal device 10 (map information or the like including location information (such as an address of the location)). This data recording unit 16 is configured by using a hard disk (not shown) as an external recording device, for example. However, instead of the hard disk or together with the hard disk, other arbitrary recording mediums including a magnetic recording medium such as a magnetic disk or an optical recording medium such as a DVD and a Blu-ray disk can be used (it is assumed that the same is applied to a data recording unit 23 of the management server 20 described later).

### (Configuration - management server)

Subsequently, the configuration of the management server 20 will be described. This management server 20 is a device for extracting a target physician who is a physician appropriate for a patient on the basis of the information acquired from the terminal device 10. As illustrated in Fig. 1, the management server 20 comprises a communication unit 21, the control unit 22, and the data recording unit 23. Note that, since this management server 20 can be configured by a known server installed in a superintendent's office, for example, detailed description thereof will be omitted.

### (Configuration - management server - communication unit)

The communication unit 21 is communication unit for communicating with the terminal device 10 via the network 2.

### (Configuration - management server - control unit)

The control unit 22 is control unit for controlling the management server 20, and is matching unit for matching a patient and a physician with each other on the basis of patient disease condition information acquired by a patient disease condition acquisition unit 22a (described later) and physician information stored in the data recording unit 23. The phrase "matching the patient and the physician with each other" unit specification of a combination of the patient and the physician. This combination does not have to be one-to-one but includes specification of a combination of a single patient and a plurality of physicians, for example. Here, the control unit 22 specifically comprises a patient disease condition acquisition unit 22a, a patient attribute acquisition unit 22b, a physician attribute acquisition unit 22c, a matching degree calculation unit 22d, and a physician extraction unit 22e in terms of functional concepts.

The patient disease condition acquisition unit 22a is patient disease condition acquisition unit for acquiring patient disease condition information specifying disease condition of a patient. The patient attribute acquisition unit 22b is patient attribute acquisition unit for acquiring patient attribute information specifying an attribute of a patient. The physician attribute acquisition unit 22c is physician attribute acquisition unit for acquiring physician attribute information specifying an attribute of a physician. The matching degree calculation unit 22d is matching degree calculation unit for calculating a matching degree indicating a degree for the physician to be suitable for medical treatment of the patient on the basis of the patient attribute information acquired by the patient attribute acquisition unit 22b and the physician attribute information acquired by the physician attribute acquisition unit 22c. The physician extraction unit 22e is physician extraction unit for extracting information relating to a target physician who is a physician suitable for the medical treatment of the patient on the basis of the patient disease condition information acquired by the patient disease condition acquisition unit 22a and physician evaluation information stored in a physician evaluation DB 23b (described later). Note that specific processing executed by each unit configuring the control unit 22 will be described later.

### (Configuration - management server - data recording unit)

The data recording unit 23 is data recording unit for recording a program and various types of data required for an operation of the management server 20, and also is physician information storage unit for storing physician information relating to physicians. The "physician information" includes any information relating to the physician, but as will be described later, includes physician evaluation information specifying evaluation of a physician and physician attribute information specifying an attribute of a physician, for example. Specifically, the data recording unit 23 comprises an ICD disease name DB 23a, a physician evaluation DB 23b, a physician attribute DB 23c, and a target physician DB 23d.

### (Configuration - management server - data recording unit - ICD disease name DB)

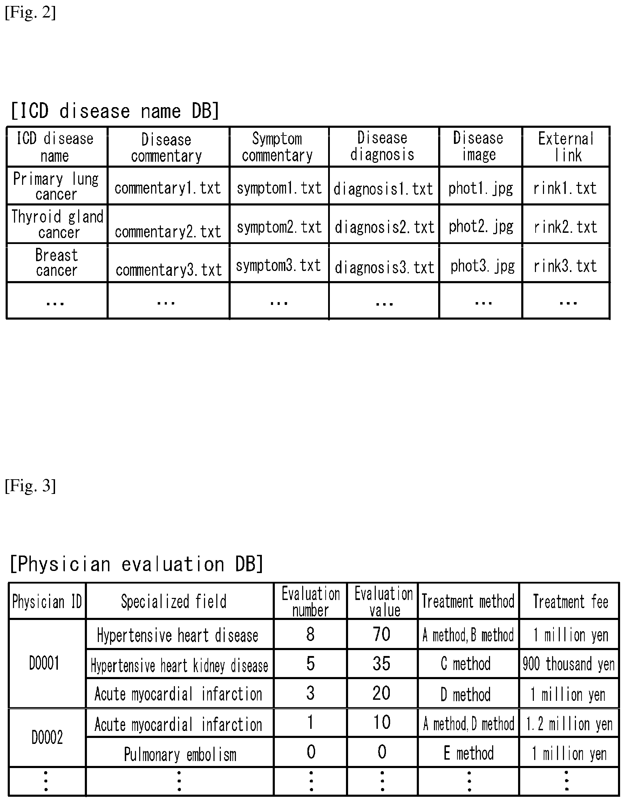

The ICD disease name dB 23a is ICD disease name information storage unit storing information for specifying an ICD disease name and disease information specifying details of the disease corresponding to the ICD disease name by associating them with each other. Here, the "disease information" is information relating to the disease and is a concept including disease commentary information, symptom commentary information, disease diagnosis information, disease image information and external link information, for example. Fig. 2 is a view illustrating a configuration example of the ICD disease name DB 23a. As illustrated in Fig. 2, the ICD disease name DB 23a is configured by associating information corresponding to an item "ICD disease name", information corresponding to an item "disease commentary", information corresponding to a "symptom commentary", information corresponding to an item "disease diagnosis", information corresponding to an item "disease image", and information corresponding to an item "external link" with each other. Here, the information corresponding to the item "ICD disease name" is ICD disease name specifying information specifying the ICD disease name, and "primary lung cancer" or the like is applicable. This "ICD disease name" unit a disease name defined in accordance with International Classification of Diseases. Moreover, the information corresponding to the item "disease commentary" is disease commentary information indicating commentary explaining contents of the disease, and a file of disease commentary information "commentary1. txt" or the like is applicable. Note that, in Fig. 2, as the information corresponding to the disease commentary information, not the file itself but a file name "commentary 1. txt" is described for convenience of illustration, and only the file name is described also for the symptom commentary information, disease diagnosis information, and disease image information which will be described later. The information corresponding to the item "symptom commentary" is symptom commentary information indicating commentary explaining contents of a symptom, and a file of symptom commentary information (file name "symptom1.txt") or the like is applicable. The information corresponding to the item "disease diagnosis" is disease diagnosis information indicating a diagnosis result of the disease, and a file of disease diagnosis information (file name "diagnosis1.txt") or the like is applicable. The information corresponding to the item "disease image" is disease image information indicating an image relating to the disease, and a file of the disease image information (file name "phot1.jpg") or the like is applicable. The information corresponding to the item "external link" is external link information specifying a link of a site on the Internet handling the contents of the diseases, and an external link information (file name "rink1.txt") or the like is applicable. Regarding the storage format for these data, the disease commentary information, symptom commentary information, disease diagnosis information, and external link information are stored in a text format, and the disease image information is stored in an image format of JPEG, PNG, GIF and the like.

### (Configuration - management server - data recording unit - physician evaluation DB)

The physician evaluation DB 23b in Fig. 1 is physician evaluation storage unit storing physician evaluation information specifying evaluation specific to a physician. Fig. 3 is a view illustrating a configuration example of the physician evaluation DB 23b. As illustrated in Fig. 3, the physician evaluation DB 23b is configured by associating information corresponding to an item "physician ID", information corresponding to an item "specialized field", information corresponding to an item "evaluation number", information corresponding to an item "evaluation value", information corresponding to an item "treatment method", and information corresponding to an item "treatment fee" with each other. Here, the information corresponding to the item "physician ID" is information for uniquely identifying a physician and in this embodiment, identification information configured by combining an alphabet D and four-digit numbers is stored. The information corresponding to the item "specialized field" is information uniquely specifying a specialized field of each physician. The specific content thereof is arbitrary, but in this embodiment, an ICD disease name such as "hypertensive heart disease", "hypertensive heart kidney disease", "acute myocardial infarction" or the like is stored. However, the information is not limited to the ICD disease name but may be general disease names such as "cancer", "pneumonia" and the like as long as such specialized field can be specified. The information corresponding to the item "evaluation number" is the number of persons who made evaluation for each specialized field of the physician (hereinafter referred to as an evaluator), and numeral values such as "8", "5", "3" or the like are stored. The information corresponding to the item "evaluation value" expresses evaluation for each specialized field of a physician in a numerical value and is a value calculated on the basis of the evaluation made by an evaluator for each specialized field of the physician. A determination method of this evaluation value is arbitrary and in this embodiment, for example, capability in a specialized field of each physician is evaluated subjectively by each evaluator in terms of any one of three-stage scores of "worthy of respect" = 10 points, "very good" = 5 points, "good" = 0 points, and a total of the evaluation scores by all the evaluators is calculated as an evaluation value. Note that, not being limited to the evaluation method as above, an evaluator may evaluate each specialized field of a physician by a score from 0 to 10 points and a total sum of the evaluation scores of all the evaluators may be calculated as an evaluation value. The information corresponding to the item "treatment method" is information specifying a specific treatment method of the disease corresponding to a specialized field of each physician, and a specific treatment method such as "A method", "B method" or "C method" is stored. The information corresponding to the item "treatment fee" is information specifying a treatment fee borne by a patient in treatment of the disease corresponding to a specialized field of each physician, and information indicating specific amount of money such as "1.2 million yen", "1 million yen" or "900 thousand yen".

### (Configuration - management server - data recording unit - physician attribute DB)

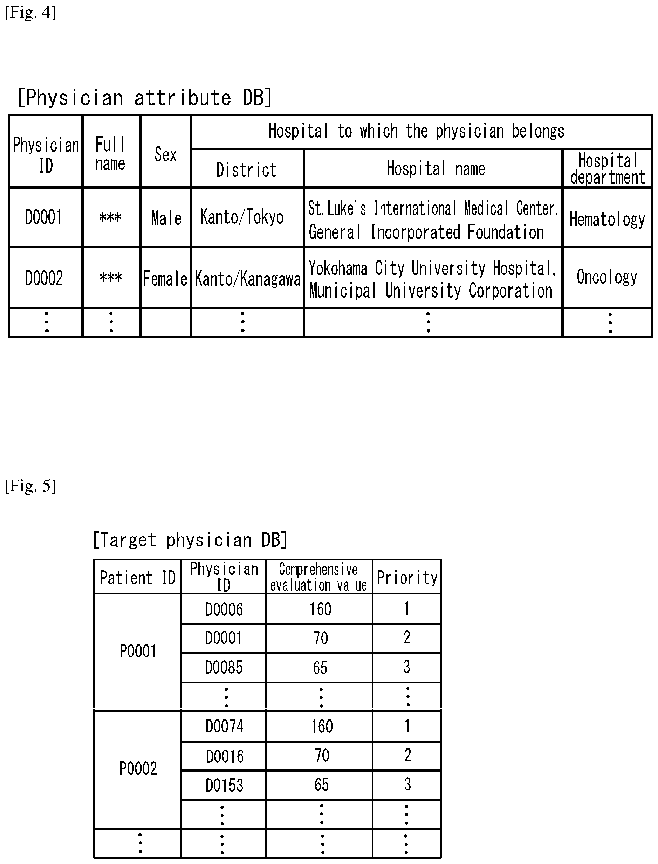

The physician attribute DB 23c in Fig. 1 is physician attribute storage unit storing the physician attribute information specifying an attribute of a physician. Fig. 4 is a view illustrating a configuration example of the physician attribute DB 23c. As illustrated in Fig. 4, the physician evaluation DB 23b is configured by associating information corresponding to the item "physician ID", information corresponding to an item "name", information corresponding to an item "sex", and information corresponding to an item "hospital to which the physician belongs" with each other. Here, the information corresponding to the item "physician ID" is information uniquely identifying a physician and is the same information as the information stored as the information corresponding to the item "physician ID" of the aforementioned physician evaluation DB 23b. The information corresponding to the item "name" is information indicating the name of each physician. The information corresponding to the item "sex" is information indicating the sex of each physician. The information corresponding to the item "hospital to which the physician belongs" is information relating to a hospital to which each physician belongs, and is configured by associating information corresponding to a sub-item "district", information corresponding to a sub-item "hospital name", and information corresponding to a sub-item "hospital department" with each other. Here, the information corresponding to the sub-item "district" is information specifying a district of a hospital to which each physician belongs, and is information indicating a name of a district and a prefecture such as "Kanto/Tokyo", "Kanto/Kanagawa" and the like. The information corresponding to the sub-item "hospital name" is information specifying a name of a hospital to which each physician belongs, and information indicating a hospital name such as "St. Luke's International Medical Center, General Incorporated Foundation" and "Yokohama City University Hospital, Municipal University Corporation" is stored. The information corresponding to the sub-item "hospital department" is information specifying a name of a hospital department to which each physician belongs, and information indicating a name of the hospital department such as "hematology" and "oncology" is stored.

### (Configuration - management server - data recording unit - target physician DB)

The target physician DB 23d in Fig. 1 is target physician information storage unit storing information relating to a target physician extracted by the physician extraction unit 22e. Fig. 5 is a view illustrating a configuration example of the target physician DB 23d. As illustrated in Fig. 5, the target physician DB 23d is configured by associating information corresponding to an item "patient ID", information corresponding to an item "physician ID", information corresponding to an item "comprehensive evaluation value", and information corresponding to an item "priority" with each other. Here, the information corresponding to the item "patient ID" is information for uniquely identifying a patient and in this embodiment, identification information configured by combining an alphabet P and four-digit numbers is stored. The information corresponding to the item "physician ID" is information for uniquely identifying a physician and is information similar to that described in the aforementioned physician evaluation DB 23b. The information corresponding to the item "comprehensive evaluation value" is a value which is an index for specifying a priority which will be described later, and is a value calculated on the basis of the aforementioned evaluation value as well as patient attribute information and physician attribute information or the like which will be described later. The information corresponding to the item "priority" is a number ranking physicians suitable for a patient and is numbered in order from a higher value of the comprehensive evaluation, for example. The information corresponding to the item "physician ID" and the item "comprehensive evaluation value" is sorted in order from a higher priority of a corresponding record (order from a lower number).

### (Processing)

Subsequently, processing executed by the medical system 1 configured as above will be described.

### (Processing - physician evaluation DB creation processing)

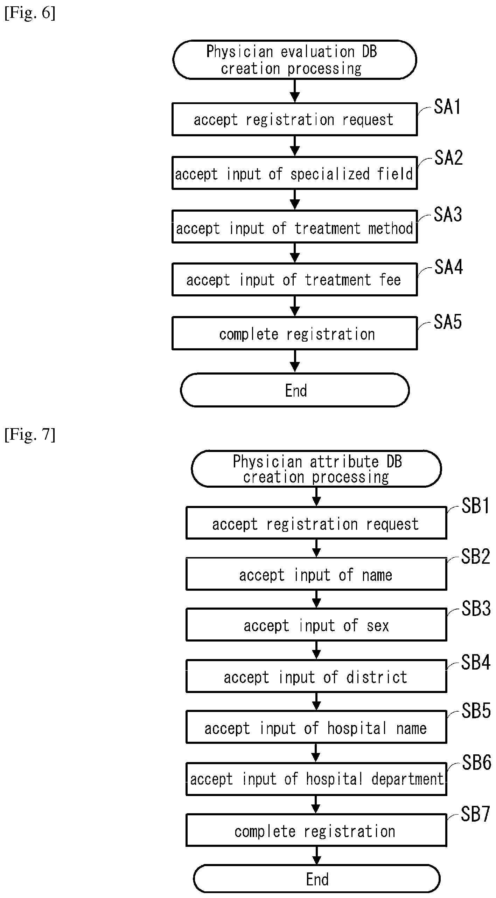

First, physician evaluation DB creation processing will be described. This physician evaluation DB creation processing is processing for creating the physician evaluation DB 23b illustrated in Fig. 3. Roughly speaking, this physician evaluation DB 23b is created by conducting inputs of each physician through an input form on the WEB and then collecting the information relating to the evaluation of the physician input by each physician (hereinafter referred to as physician evaluation information). Fig. 6 is a flowchart of the physician evaluation DB creation processing (hereinafter, step is abbreviated as "S" in description of each processing).

First, as illustrated at SA1, the control unit 22 of the management server 20 accepts a registration request of the physician evaluation information. A registration request is made by a click by a physician on a new registration icon displayed on the display 13 of the terminal device 10, for example. When the registration request is accepted as above, a physician ID specific to the physician is determined at random, and the routine proceeds to SA2.

At SA2, the control unit 22 of the management server 20 accepts an input of a specialized field from the physician. The input can be made by listing a plurality of ICD disease names on the display 13 of the terminal device 10 and selecting the ICD disease name of the specialized field of the physician among them by a click, for example. A plurality of the ICD disease names may be selected.

At SA3, the control unit 22 of the management server 20 accepts an input of a treatment method from a physician. A plurality of treatment methods corresponding to the ICD disease name selected by the physician at SA2 is listed on the display 13 of the terminal device 10, for example. The treatment methods are listed by referring to a treatment method table (not shown) storing the ICD disease name and the treatment method in association with each other. Then, an input can be made by the physician selecting the treatment method of the physician's specialty in the treatment methods listed as above by a click. A plurality of the treatment methods may be selected.

At SA4, the control unit 22 of the management server 20 accepts an input of a treatment fee from a physician. For example, an input form of a treatment fee may be displayed on the display 13 of the terminal device 10 for each ICD disease name selected by the physician at SA2, and the physician may input a specific amount in this input form.

At SA5, the control unit 22 of the management server 20 completes registration and stores the information corresponding to the item "physician ID", the information corresponding to the item "specialized field", the information corresponding to the item "treatment method", and the information corresponding to the item "treatment fee" in the physician evaluation DB 23b illustrated in Fig. 3 on the basis of each piece of information input in the processing at SA1 to SA4. For both the information corresponding to the item "evaluation number" and the information corresponding to the item "evaluation value", "0" is stored as an initial value. When the registration is completed as above, a specific physician ID is delivered to a physician, and an account is created and a WEB page displaying various types of information relating to the physician's own account (information such as specialized field and treatment method (if information such as the name and sex of the physician and a hospital name of the hospital to which the physician belongs is registered in physician attribute DB creation processing which will be described later, these types of information are also included)) is generated. Then, the physician can view and edit the information on the WEB page by inputting the physician's own ID and a password set in advance and the like through a login form. The physician evaluation DB creation processing is completed as above.

### (Processing - physician attribute DB creation processing)

Subsequently, the physician attribute creation processing will be described. This physician attribute DB creation processing is processing for creating the physician attribute DB 23c illustrated in Fig. 4. Roughly speaking, this physician attribute DB 23c is created by conducting inputs of each physician through an input form on the WEB and then collecting information input by each physician (hereinafter referred to as physician attribute information). Fig. 7 is a flowchart of the physician attribute DB creation processing.

At SB1, the control unit 22 of the management server 20 accepts a registration request of the physician attribute information. The registration request is made by conducting inputs of the physician's physician ID, a password and the like by a physician on a login form displayed on the display 13 of the terminal device 10 so as to log in the physician's own account and by a click in a "profile edit" icon displayed on the display 13, for example. When the registration request is accepted as above, the routine proceeds to SB2.

At SB2, the control unit 22 of the management server 20 accepts an input of the name from the physician. An input form for inputting a name may be displayed on the display 13 of the terminal device 10, and the physician may input his/her own name in this input form, for example.

At SB3, the control unit 22 of the management server 20 accepts an input of the sex from the physician. A check column for male or female may be provided on the display 13 of the terminal device 10 and the physician may conduct an input by checking the check column applicable to his/her own sex, for example.

At SB4, the control unit 22 of the management server 20 receives an input of a district where a hospital to which the physician belongs is located from the physician. An input form for inputting a district may be displayed on the display 13 of the terminal device 10, and the physician may input the district of the hospital to which the physician belongs in this input form, for example.

At SB5, the control unit 22 of the management server 20 accepts an input of the name of a hospital to which the physician belongs from the physician. An input form for inputting the name of the hospital may be displayed on the display 13 of the terminal device 10, and the physician may input the name of the hospital to which the physician belongs in this input form, for example.

At SB6, the control unit 22 of the management server 20 accepts an input of a hospital department to which the physician belongs from the physician. An input form for inputting a hospital department may be displayed on the display 13 of the terminal device 10 and the physician may input the hospital department to which the physician belongs in this input form, for example.

At SB7, the control unit 22 of the management server 20 completes the registration and stores the information corresponding to the item "physician ID", the information corresponding to the item "name", the information corresponding to the item "sex", the information corresponding to the sub-item "district", the information corresponding to the sub-item "hospital name", and the information corresponding to the sub-item "hospital department" of the physician attribute DB 23c illustrated in Fig. 4 on the basis of each piece of information input in the processing at SB1 to SB6. The physician attribute DB creation processing is completed as above.

### (Processing - physician evaluation processing)

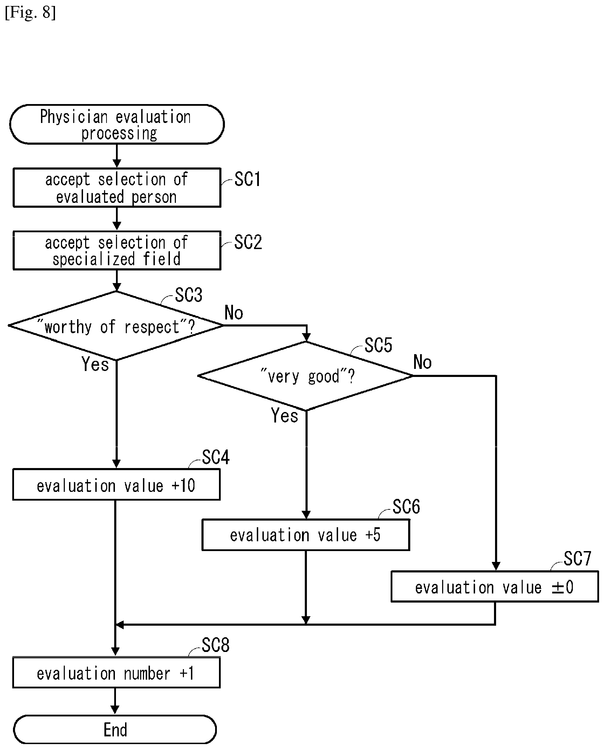

Subsequently, the physician evaluation processing will be described. The physician evaluation processing is processing for updating the information corresponding to the item "evaluation number" and the information corresponding to the item "evaluation value" in the physician evaluation DB 23b illustrated in Fig. 3. Fig. 8 is a flowchart of the physician evaluation processing. A person who executes such physician evaluation processing is arbitrary as long as the person knows the physician to be evaluated, and may include the physicians of the same profession as the physician to be evaluated, those working for the same office, and patients, for example. However, in this embodiment, it is assumed that only the physicians owning the physician ID by executing the physician evaluation DB creation processing illustrated in the aforementioned Fig. 6 can execute the physician evaluation processing. In the following, a physician to conduct evaluation is called an "evaluator" and the physician to be evaluated is called an "evaluated person" in description as necessary.

At SC1, the control unit 22 of the management server 20 accepts selection of the physician to be an evaluated person. An acception method of such selection of the physician is arbitrary, and a physician belonging to the same hospital as that of the evaluated person may be automatically selected as an evaluated person on the basis of the physician attribute information, for example. However, in this embodiment, the selection is accepted by the following method. First, when the physician inputs his/her own physician ID, a password and the like in the login form displayed on the display 13 of the terminal device 10 and logs in his/her account, the WEB page of his/her account is displayed on the display 13 of the terminal device 10, and information of the other plurality of physicians (name and hospital name to which the physician belongs, for example) is listed on this WEB page. Then, by clicking the information of the listed physician, the page moves to the WEB page of the physician's account, and by clicking the "evaluate this physician" icon displayed on the WEB page, the evaluated person is selected.

At SC2, the control unit 22 of the management server 20 accepts selection of the specialized field of the physician to be an evaluated person. The selection is accepted by having the ICD disease name registered as the specialized field of the evaluated person displayed on the display 13 of the terminal device 10 and by having the evaluator select displayed ICD disease name, for example.

At SC3, the control unit 22 of the management server 20 determines whether the evaluation of the evaluated person by the evaluator is "worthy of respect" or not. Specifically, three check columns, that is, "worthy of respect", "very good", and "good" are displayed on the display 13 of the terminal device 10 of the evaluated person, and it is determined whether the check column of "worthy of respect" has been checked by the evaluated person or not. Then, if "worthy of respect" is checked (SC3, Yes), the routine proceeds to SC4, while if "worthy of respect" is not checked (SC3, No), the routine proceeds to SC5.

At SC4, the control unit 22 of the management server 20 adds a score (=10 points) associated with "worthy of respect" to the information which is a record corresponding to the physician ID of the evaluated person and corresponding to the item "evaluation value" in the record corresponding to the specialized field selected by the evaluator at the aforementioned SC2 in the physician evaluation DB 23b illustrated in Fig. 3. Specifically, an evaluation information table (not shown) storing the information indicating an evaluation content ("worthy of respect", "very good", "good" and the like) and the information indicating the score ("10 points", "5 points", and "0 points") associated with each other is referred to, and the score stored correspondingly to the evaluation content selected by the evaluator is added.

At SC5, the control unit 22 of the management server 20 determines whether the evaluation by the evaluator of the evaluated person is "very good" or not. Specifically, in the aforementioned three check columns, it is determined whether the check column of "worthy of respect" has been checked by the evaluated person or not and then, if "very good" is checked (SC5, Yes), the routine proceeds to SC6, while if "very good" is not checked (SC5, No), the routine proceeds to SC7.

At SC6, the control unit 22 of the management server 20 adds 5 points to the item "evaluation value" in the record corresponding to the physician ID of the evaluated person. A specific procedure of this addition is executed by the method similar to that at the aforementioned SC4.

At SC7, that is, when neither of the check column of "worthy of respect" or the check column of "very good" is checked, that is, when the check column of "good" is checked, the control unit 22 of the management server 20 does not add anything to the item "evaluation value" in the record corresponding to the physician ID of the evaluated person. By completing the processing at SC4, SC6 or SC7, the evaluation value is accumulated, and the routine proceeds to SC8 after that.

At SC8, the control unit 22 of the management server 20 increases the item "evaluation number" in the record corresponding to the physician ID of the evaluated person by 1. As a result, the number of evaluators who made evaluation of the specialized field of the evaluated person is accumulated. The physician ID of the evaluator who made the evaluation of the evaluated person is preferably stored in the data recording unit 23 of the management server 20 so that the same evaluator cannot evaluate the same evaluated person a plurality of times. The physician evaluation processing is completed as above.

### (Processing - target physician output processing)

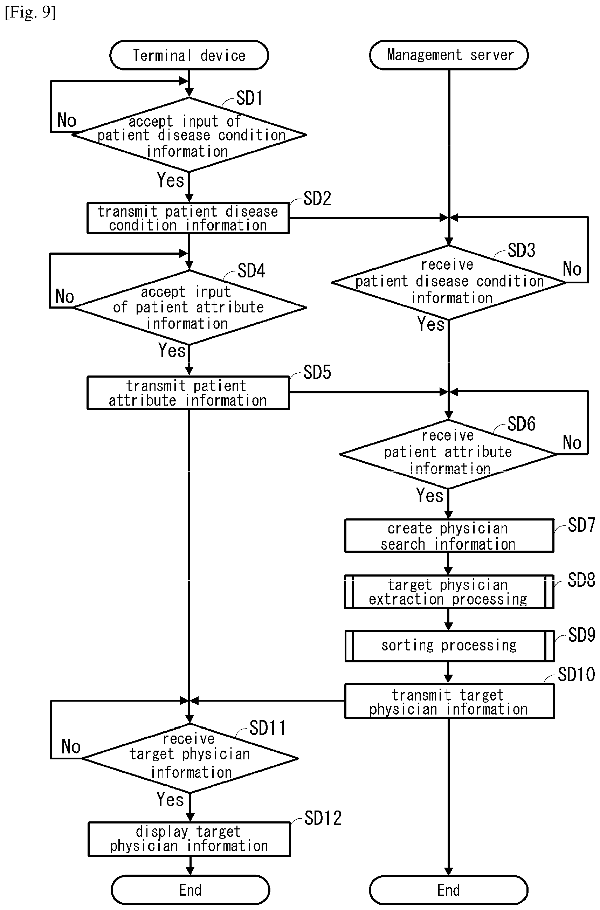

Subsequently, the target physician output processing will be described. This target physician output processing is processing for outputting information relating to a target physician appropriate for a patient to the patient when the patient transmits patient disease condition information and patient attribute information. Fig. 9 is a flowchart of the target physician output processing. Timing when this target physician output processing is executed is arbitrary and it is executed at timing when the terminal device 10 and the management server 20 are powered on, for example.

First, at SD1, the control unit 15 of the terminal device 10 determines whether an input of the patient disease condition information has been accepted or not. A specific method of this determination is arbitrary, and a list of the ICD disease names is displayed on the display 13 of the terminal device 10, and it is determined that the input has been accepted when the patient selects an ICD disease name indicating the patient's disease in this list, for example. Then, it stands by until the input is accepted (SD1, No), and when the input is accepted (SD1, Yes), the routine proceeds to SD2.

At SD2, the control unit 15 of the terminal device 10 transmits the patient disease condition information input at SD1 to the management server 20 through the communication unit 11 of the terminal device 10.

At SD3, the patient disease condition acquisition unit 22a of the management server 20 determines whether the patient disease condition information has been received from the terminal device 10. Then, it stands by until reception (SD3, No), and upon reception (SD3, Yes), the routine proceeds to SD6.

At SD4, the control unit 15 of the terminal device 10 determines whether the input of the patient attribute information has been accepted or not. A specific method of this determination is arbitrary, and an input form for inputting the "age", "sex", "location", and "budget" as the patient attribute information is displayed on the display 13 of the terminal device 10, for example, and it is determined that the input has been accepted by the fact that an input is made in this input form. The patient may skip the input in a part of or the whole of this input form. Then, it stands by until the input is accepted (SD4, No), and when the input has been accepted (SD4, Yes), the routine proceeds to SD5.

At SD5, the control unit 15 of the terminal device 10 transmits the patient attribute information input at SD4 to the management server 20 through the communication unit 11 of the terminal device 10.

At SD6, the patient attribute acquisition unit 22b of the management server 20 determines whether the patient attribute information has been received from the terminal device 10 or not. Then, it stands by until reception (SD6, No), and upon reception (SD6, Yes), the routine proceeds to SD7.

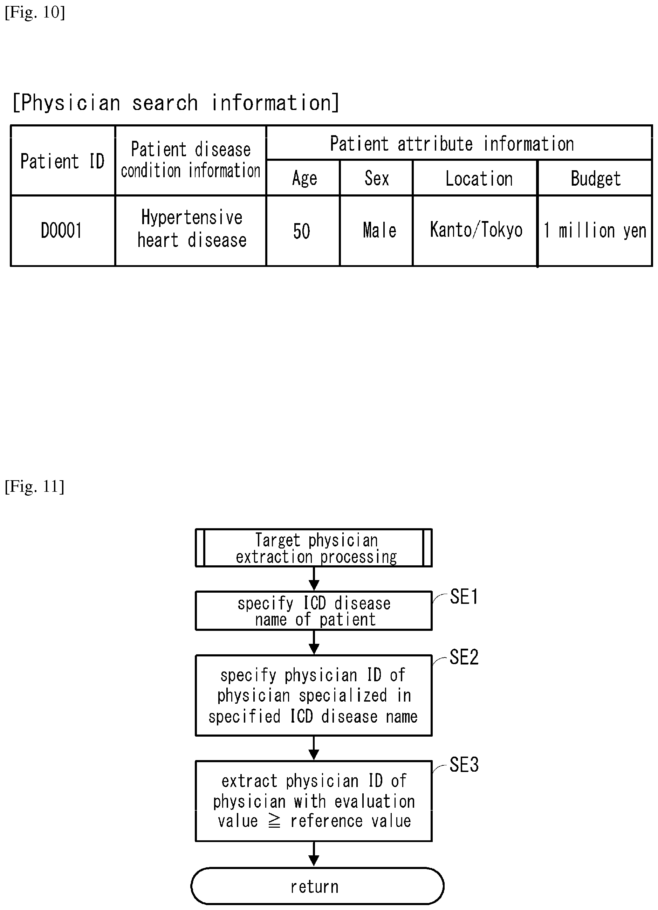

At SD7, the control unit 22 of the management server 20 creates physician search information on the basis of the patient disease condition information received at SD3 and the patient attribute information received at SD6. Here, Fig. 10 is a view illustrating a configuration example of the physician search information. This physician search information is configured by associating information corresponding to the item "patient ID", information corresponding to the item "patient disease condition information", and information corresponding to the item "patient attribute information" with each other. The information corresponding to the item "patient ID" is a patient ID of the patient who made a physician search. The information corresponding to the item "patient disease condition information" is information for uniquely specifying the disease condition of the patient and the ICD disease name of the patient input in the processing at SD1 is stored. The information corresponding to the item "patient attribute information" is information for uniquely specifying the attribute of the patient, and the attribute of the patient input in the processing at SD4 is stored. Specifically, the information corresponding to the sub-item "age", the information corresponding to the sub-item "sex", the information corresponding to the sub-item "location", and the information corresponding to the sub-item "budget" are associated with each other and stored, and the information relating to the age, sex, location and budget of the patient is stored, respectively.

### (Processing - target physician output processing - target physician extraction processing)

Returning to Fig. 9, at SD8, the physician extraction unit 22e of the management server 20 executes target physician extraction processing. In the following, the target physician extraction processing will be described. Fig. 11 is a flowchart of the target physician extraction processing. This target physician extraction processing is processing for extracting a target physician who is a physician appropriate for the patient on the basis of the physician search information.

First, at SE1, the physician extraction unit 22e specifies the ICD disease name of the patient. Specifically, the information corresponding to the item "patient disease condition information" in the physician search information in Fig. 10 created at SD7 of the target physician output processing illustrated in Fig. 9 is specified as the ICD disease name of the patient ("hypertensive heart disease" in Fig. 10).

At SE2, the physician extraction unit 22e specifies the physician ID of the physician specialized in the ICD disease name of the patient specified at SE1. Specifically, the information corresponding to the item "specialized field" of the physician evaluation DB 23b illustrated in Fig. 3 is referred to, and the physician ID of the record storing the ICD disease name specified at SE1 is specified. In Fig. 3, there is "D0001" as the physician ID of the record storing the hypertensive heart disease which is the ICD disease name of the patient, for example. Similarly, all the physician IDs of the physicians specialized in the hypertensive heart disease are specified.

At SE3, the physician extraction unit 22e extracts the physician ID having the evaluation value larger than a reference value in all the physician IDs specified at SE2. Here, the "reference value" is a reference set so that a physician with a low evaluation is not output for the patient and is stored in the data recording unit 23 of the management server 20 in advance, for example. As the reference value, an arbitrary value such as "10", "20" or "30" is set. When all the physicians are to be output regardless of the evaluation, it is only necessary to set the reference value to "0". Extraction of the physician ID is completed as above, and returning to the target physician output processing in Fig. 9, the routine proceeds to SD9.

### (Processing - target physician output processing - sorting processing)

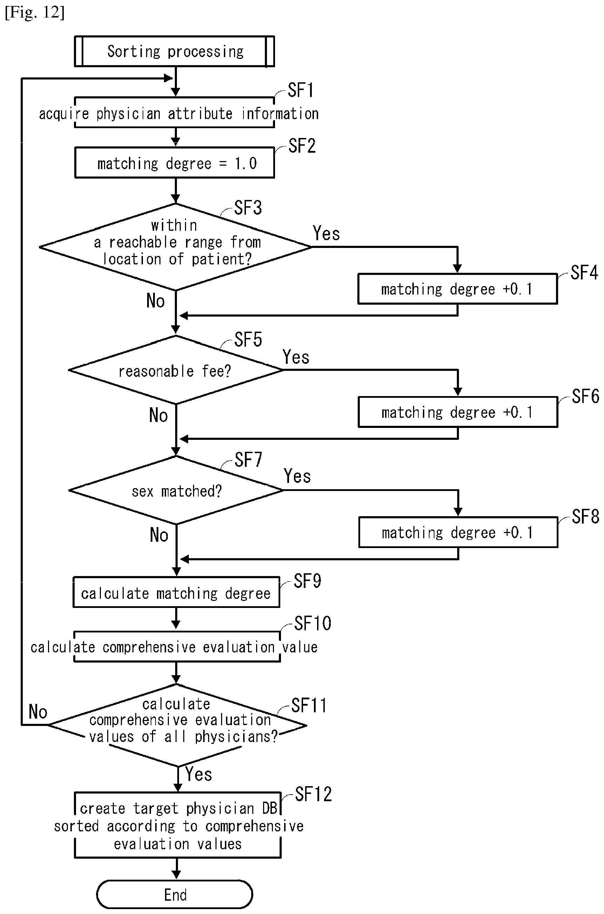

At SD9, the control unit 22 of the management server 20 executes sorting processing. This sorting processing is processing of sorting all the physician IDs extracted at SE3 in an order of physicians appropriate for a patient. Fig. 12 is a flowchart of the sorting processing. Here, in this sorting processing, roughly speaking, one physician ID is extracted from the physician IDs extracted at SE3 in Fig. 11, a comprehensive evaluation value (described later) of this physician ID is calculated, and then, another physician ID is extracted from the physician IDs extracted at SE3 in Fig. 11, the comprehensive evaluation value of this physician ID is calculated, and the similar processing is repeated after that, whereby the comprehensive evaluation values of all the physician IDs extracted at SE3 in Fig. 11 are calculated. In the following, the physician indicated by the physician ID for whom the comprehensive evaluation value is calculated is called a "calculation target physician" in description.

First, at SF1, the matching degree calculation unit 22d of the management server 20 acquires the physician attribute information relating to the calculation target physician. Specifically, the physician attribute DB 23c illustrated in Fig. 4 is referred to, and a record storing the physician ID extracted at SE3 is acquired as information corresponding to the item "physician ID".

At SF2, the matching degree calculation unit 22d of the management server 20 sets the matching degree of the calculation target physician to "1.0". This matching degree is a value which is a reference for specifying matching between the attribute of the patient and the attribute of the physician, and an initial value is set to "1.0" as above.

At SF3, the matching degree calculation unit 22d of the management server 20 determines whether the hospital to which the calculation target physician belongs is within a reachable range from the location of the patient. A specific method of this determination is arbitrary, but it may be determined to be within the reachable range if the location of the hospital to which the calculation target physician belongs stored as the information corresponding to the item "district" in the physician attribute DB 23c illustrated in Fig. 4 matches the location of the patient stored as the information corresponding to the item "location" in the physician search information illustrated in Fig. 10. Alternatively, it may be determined to be within the reachable range if time for moving from the location of the patient to the location of the hospital to which the calculation target physician belongs is within a predetermined time (2 hours, for example). This predetermined time may be set arbitrarily, but the age of the patient may be considered in setting and if the age of the patient is old, for example, the predetermined time may be set shorter by considering that moving for a long time is difficult. Moreover, a measuring method of the moving time is arbitrary and can be measured by using a known route search application, for example. Then, if it is within the reachable range (SF3, Yes), the routine proceeds to SF4, while if it is not within the reachable range (SF3, No), the routine proceeds to SF5.

At SF4, the matching degree calculation unit 22d of the management server 20 assumes that the calculation target physician and the patient geographically match well and adds 0.1 to the matching degree. A specific point to be added at this time is arbitrary, and 0.2 or 0.3 may be added, for example. Moreover, the value may be different from a value to be added at SF6 or SF8 which will be described later.

At SF5, the matching degree calculation unit 22d of the management server 20 determines whether the fee of the calculation target physician is reasonable for the patient. A specific method of this determination is arbitrary, but it may be determined to be reasonable if the treatment fee of the calculation target physician stored as the information corresponding to the item "treatment fee" in the physician evaluation DB 23b illustrated in Fig. 3 is contained in the budget of the patient stored as the information corresponding to the item "budget" in the physician search information illustrated in Fig. 10, for example. Alternatively, it may be determined to be reasonable if a difference between the budget of the patient and the treatment fee of the calculation target physician is a predetermined amount (200 thousand yen, for example) or less. If it is reasonable (SF5, Yes), the routine proceeds to SF6, while if it is not reasonable (SF5, No), the routine proceeds to SF7.

At SF6, the matching degree calculation unit 22d of the management server 20 determines that the calculation target physician and the patient match each other well in view of a fee and adds 0.1 to the matching degree. A specific point to be added at this time is arbitrary, and 0.2 or 0.3 may be added, for example.

At SF7, the matching degree calculation unit 22d of the management server 20 determines whether the sex of the calculation target physician matches the sex of the patient. A specific method of this determination is arbitrary, but by comparing the sex of the calculation target physician stored as the information corresponding to the item "sex" in the physician attribute DB 23c illustrated in Fig. 4 with the sex of the patient stored as the information corresponding to the item "sex" in the physician search information illustrated in Fig. 10 and if they match each other, it may be determined that the sex of the both match each other. If they match each other (SF7, Yes), the routine proceeds to SF8, while if not (SF7, No), the routine proceeds to SF9.

At SF8, the matching degree calculation unit 22d of the management server 20 determines that the calculation target physician and the patient match each other well in view of sex and adds 0.1 to the matching degree. A specific point to be added at this time is arbitrary, and 0.2 or 0.3 may be added, for example.

At SF9, the matching degree calculation unit 22d of the management server 20 calculates a final matching degree of the calculation target physician after the processing at the aforementioned SF1 to SF8. Specifically, a value of "1.0" to "1.3" is calculated as the matching degree in the aforementioned example.

At SF10, the matching degree calculation unit 22d of the management server 20 calculates the comprehensive evaluation value of the calculation target physician. This comprehensive evaluation value is information for specifying the comprehensive evaluation of the calculation target physician for the patient. The specific unit for calculating the comprehensive evaluation value is arbitrary but in this embodiment, it is calculated on the basis of the information corresponding to the item "evaluation value" of the calculation target physician in the physician evaluation DB 23b illustrated in Fig. 3 and the matching degree calculated at SF9 in Fig. 12. Specifically, the comprehensive evaluation value is calculated by multiplying the matching degree by the evaluation value. However, such calculation method is not limiting, and calculation may be made by considering elements other than the evaluation value or the matching degree, for example. The information corresponding to the item "evaluation number" of the calculation target physician in the physician evaluation DB 23b illustrated in Fig. 3 is referred to, and the comprehensive evaluation value may be further increased by assuming that a physician with more evaluation number has higher reputation, for example.

At SF11, the matching degree calculation unit 22d of the management server 20 determines whether the comprehensive evaluation values of all the physician IDs extracted at SE3 in Fig. 11 have been calculated or not, and if they have not been calculated (SF11, No), the routine returns to SF1, and the processing at SF1 to SF11 is repeated after that by assuming the physician for whom the comprehensive evaluation value has not been calculated yet as a calculation target physician. If the comprehensive evaluation values of all the physician IDs extracted at SE3 in Fig. 11 have been calculated (SF11, Yes), the routine proceeds to SF12.

At SF12, the matching degree calculation unit 22d of the management server 20 creates the target physician DB 23d illustrated in Fig. 5 listing the physician IDs in the order from those with higher comprehensive evaluation value calculated in the processing illustrated in SF1 to SF11. For the patient with the patient ID "P0001", the physicians with the physician IDs "D0006", "D0001", and "D0085" are listed in the order from those with the higher priority (that is, the order from those with the higher comprehensive evaluation value). For the patient with the patient ID "P0002", the physicians with the physician IDs "D0074", "D0016", and "D0153" are listed in the order from those with the higher priority. The sorting processing is finished as above, and the routine returns to the target physician output processing illustrated in Fig. 9.

Subsequently, at SD10, the control unit 22 of the management server 20 transmits the information of the record corresponding to the patient ID of the patient at a transmission destination in the target physician information stored in the target physician DB 23d illustrated in Fig. 5 to the terminal device 10 of the patient. At this time, the ID uniquely specifying the terminal device 10 of the patient is added to the target physician information to be transmitted in transmission.

At SD11, the control unit 15 of the terminal device 10 determines whether the target physician information has been received or not. This determination method is arbitrary, and for example, whether the ID of the terminal device 10 matches the ID added to the target physician information on the terminal device 10 side may be determined, and then if they match, it may be determined that the target physician information has been received. Then, the control unit 15 of the terminal device 10 stands by until the target physician information has been received (SD11, No), and if it is received (SD11, Yes), the routine proceeds to SD12.

At SD12, the control unit 15 of the terminal device 10 displays the target physician information received at SD11 on the display 13 of the terminal device 10. As described above, by displaying the target physician information listing the physicians in the order from those with the higher comprehensive evaluation value, the patient can easily recognize an appropriate physician for himself/herself. Clicking the ID of each physician in the target physician information displayed on the display 13 may make the detailed information relating to the clicked physician to be viewable. For example, the information relating to the name and sex of the physician as well as the hospital to which the physician belongs stored in the physician attribute DB 23c, or the information relating to the specialized field, treatment method, and treatment fee stored in the physician evaluation DB 23b may be made viewable. Moreover, information other than them may be stored in the physician attribute DB 23c or the physician evaluation DB 23b or the like so that the information may be viewable. For example, a profile photo of the physician or a thesis written by the physician or the like may be made viewable, for example. Moreover, clicking the ICD disease name in the specialized field of the physician may make the information stored in the ICD disease name dB 23a illustrated in Fig. 2 to be referred to, and detailed information relating to the disease corresponding to the ICD disease name to be viewable. Specifically, each of the item names (disease commentary, symptom commentary, disease diagnosis, disease image, and external link) in Fig. 2 may be listed on the display 13 by clicking the ICD disease name, and the information corresponding to the item may be read from the ICD disease name DB 23a in Fig. 2 and displayed on the display 13 by clicking any one of the items. The target physician output processing is finished as above.

### (Advantageous effects of the embodiment)

As described above, according to the embodiment, since the patient and the physician are matched with each other on the basis of the disease condition information and the physician information, matching can be made on the basis of the disease condition of the patient and the information of the physician, and the patient can select a physician appropriate for his/her own disease or the physician can select an appropriate patient in accordance with his/her own techniques.

Moreover, since the information relating to a target physician is output on the basis of the patient disease condition information and the physician evaluation condition, a physician determined to be objectively excellent can be output with priority in selecting a target physician, and the patient can select a physician more appropriate for his/her own disease.

Moreover, since the evaluation of the physician in relation to a disease corresponding to the ICD disease name is stored, the evaluation of the physician relating to the internationally regulated reference can be stored, and a single integrated evaluation can be stored even for a disease having a plurality of disease names.

Moreover, since the evaluation of the physician in relation to a treatment method corresponding to the disease condition of the patient is stored, even if a plurality of different diseases can be treated by a single treatment method or the like, the evaluation of a physician can be appropriately stored.

Moreover, the matching degree indicating a degree for the physician to be suitable for medical treatment of the patient is calculated on the basis of the patient attribute information and the physician attribute information, and the target physician is extracted by considering this matching degree and thus, the target physician can be extracted on the basis of the information other than the patient's disease condition or the evaluation of the physician and the information specific to the patient and the physician, and the patient can select a physician more appropriate for a treatment the patient receives for the his/her own disease.

Moreover, since the attribute of the patient includes age, sex, a current location or a medical treatment fee of the patient, the target physician can be extracted on the basis of the specific information such as the age, sex, current location or treatment fee of the patient, and the patient can select a physician more appropriate for a medical treatment the patient receives for the his/her own disease.

Moreover, since the evaluation of the physician is determined on the basis of evaluation from another physician who has specialized knowledge, more appropriate evaluation can be stored.

### [Variation]

The embodiment according to the present invention has been described above, but specific configuration and unit of the present invention can be arbitrarily modified or improved within a range of a technical idea of each invention described in claims. Hereinafter, such variations will be described.

### (Problems to be solved and advantageous effects of invention)

First, the problems to be solved by the invention and the advantageous effects of the invention are not limited to the aforementioned contents, but the invention can solve problems not described above or can exert advantageous effects not described above, and moreover, can solve only a part of the described problems or can exert only a part of the described advantageous effects. Even if a physician appropriate for a patient cannot be selected as compared with a conventional system, for example, when unit of the invention of the present application is different from unit of the conventional system, the problem of the invention of the present application is solved.

### (Distribution and integration)

Moreover, each of the aforementioned electric constituent elements is a functional concept and does not necessarily require physical constitution as illustrated. That is, specific forms of distribution or integration of each unit are not limited to those illustrated, and the whole of or a part of them may be configured by physically or functionally distributing or integrating them by the arbitrary unit in accordance with various loads and use conditions. For example, the terminal device 10 or the management server 20 may be configured in a distributed manner by a plurality of devices, the terminal device 10 or the management server 20 may be integrated with each other, or a part of functions of either one of the terminal device 10 and the management server 20 may be provided in the other. Moreover, when distributing the units, cooperation among these units can be realized by either one or both of wired and wireless manners.

### (Shape, numeral value, structure, and time series)

Regarding the constituent elements exemplified in the embodiment or in the figures, the shape, numeral values or structures of a plurality of the constituent elements or a mutual relationship in a time series may be arbitrarily modified and improved within a range of the technical idea of the present invention.

### (Evaluation)

In this embodiment, as illustrated in Fig. 3, it is described that the evaluation of a physician relating to a specialized field of the physician is stored as the physician evaluation information, but this is not limiting, and evaluation of the physician relating to a treatment method, for example, may be stored. By storing the evaluation of the physician relating to the treatment method corresponding to the disease condition of the patient as the physician evaluation information as above, even if a plurality of different diseases can be treated by a single treatment method or the like, the evaluation of a physician can be appropriately stored.

Moreover, in this embodiment, it is described that the evaluation of a physician is determined on the basis of the evaluation from another physician, but this is not limiting, and the evaluation of a physician may be determined on the basis of the evaluation from a patient. Alternatively, the evaluation of a physician may be determined on the basis of both the evaluation from another physician and the evaluation from the patient, and an evaluation value to which a point is added by the evaluation from the physician and the evaluation value to which a point is added by the evaluation from the patient are made different values at this time, and a single evaluation value may be calculated by a total sum of the evaluation values. Alternatively, these respective evaluation values are handled separately, and a "target physician determined to be appropriate on the basis of the evaluation value from a physician" and a "target physician determined to be appropriate on the basis of an evaluation value from a patient" may be both output.

### (Evaluation value)

In this embodiment, as an evaluation method, evaluation is made by a standard of any one of three stages, that is, "worthy of respect", "very good", and "good", but this is not limiting, and the evaluation may be made by score of any from "0" to "10" as an evaluation value, for example. Alternatively, a negative evaluation may be added as the evaluation standard, and 5-stage standards with two more stages of "bad" and "very bad" added may be provided, and the evaluation with "bad" may be set to an evaluation value of "-5", and the evaluation with "very bad" to an evaluation value of "-10".

Moreover, an evaluation value of a physician may be specified by a method other than the evaluation from the others. For example, an evaluation value of a physician may be specified on the basis of a career, business experience, academic background, activities and the like by which the physician should be evaluated. As a specific example, a method of specifying an evaluation value of a physician on the basis of the activities of the physician (particularly writing of a thesis, a book and the like by the physician (hereinafter referred to as "theses") will be described below. First, information relating to theses written by a physician (contents of the theses or only titles of the theses) is made storable in the physician attribute DB 23c or the physician evaluation DB 23b. They may be storable by input by the physician himself/herself through the operation unit 12. Then, when the theses are stored in the physician attribute DB 23c or the physician evaluation DB 23b and the like, the physician is considered to have authority and the evaluation value of the specialized field corresponding to the theses may be increased by a predetermined value (+30, for example). Besides, as a method of specifying an evaluation value on the basis of the career of a physician, a period for which the physician is engaged with a disease in the specialized field as a physician may be made storable in the physician attribute DB 23c or the physician evaluation DB 23b, for example, and the evaluation value may be increased by a predetermined value in accordance with the period. In the case of 30 years or more, the evaluation value is given +100, in the case of 20 years or more, the evaluation value is given +50, and in the case of 10 years or more, the evaluation value is given +20 or the like may be set, for example.

Moreover, a co-writer of the theses may be made storable in the data recording unit 23 of the management server 20 (the physician attribute DB 23c or the physician evaluation DB 23b and the like, for example). First, when a physician who makes an input (hereinafter referred to as a "physician A") makes an input of theses through the operation unit 12 as above, for example, an item in which a co-writer is to be input is displayed on the display 13, and information for specifying another physician (hereinafter referred to as a "physician B") who is a co-writer is input in this item. Subsequently, the control unit 22 of the management server 20 associates information relating to a writer of the theses with the theses and stores them in the data recording unit 23 of the management server 20 on the basis of input contents and the like. A specific form of storage is arbitrary, but information specifying the physician B input by the physician A and the information relating to the theses may be associated and stored in the record of the physician A in the physician attribute DB 23c, for example. Alternatively, a record of the physician B in the physician attribute DB 23c may be specified on the basis of the information specifying the physician B input by the physician A, and the information specifying the physician A and the information relating to the theses are associated and stored in the record of the physician B. Alternatively, the both modes are combined, and the information specifying the respective co-writers and the information relating to the theses may be associated and stored in both the records of the physician A and the physician B. Alternatively, in a thesis information DB (not shown) associating information specifying all the writers of the theses (in this case, the physician A and the physician B) and information relating to the theses with each other for storage, the information specifying the physician A, the information specifying the physician B, and the information relating to the theses written by the physician A and the physician B may be stored. As a result, the physician B who is a co-writer can omit a labor of inputting the information relating the theses by himself/herself, and accumulation of the information relating to the theses can be streamlined.

Moreover, in this embodiment, when the comprehensive evaluation value is to be calculated, the information corresponding to the item "evaluation value" in Fig. 3 is used as it is, but this is not limiting, and an average value of the evaluation may be used. Specifically, the comprehensive evaluation value may be calculated by using a value acquired by dividing a numeral value of the information corresponding to the item "evaluation value" by a numeral value of the information corresponding to the item "evaluation number". As a result, a physician who has a smaller evaluation number but high evaluation can be extracted easily.

### (Target physician output processing)

In this embodiment, at SD12 of the target physician output processing in Fig. 9, it is described that information of a record corresponding to the patient ID of the patient as a transmission destination (that is, the physician ID, comprehensive evaluation value, and priority in Fig. 5) in the target physician information stored in the target physician DB 23d illustrated in Fig. 5 is displayed on the display 13, but the information to be displayed is not limited to such information. For example, any one of the physician ID, comprehensive evaluation value, and priority may not be displayed, information which can specify a physician (the name of a physician specified on the basis of the physician attribute DB 23c, for example) may be displayed instead of the physician ID, or all of them may be displayed.

Moreover, in this embodiment, the physicians are displayed in the order from those with the higher comprehensive evaluation value, but this is not limiting, and the physicians may be displayed in an arbitrary order or in an arbitrary display form. For example, the physicians may be classified into a plurality of classes in accordance with the comprehensive evaluation values (a first class for those with the comprehensive evaluation values within upper 20%, a second class for those with upper 20 to 40%, and a third class for the others), and the physicians may be displayed in each class.

### (Matching)

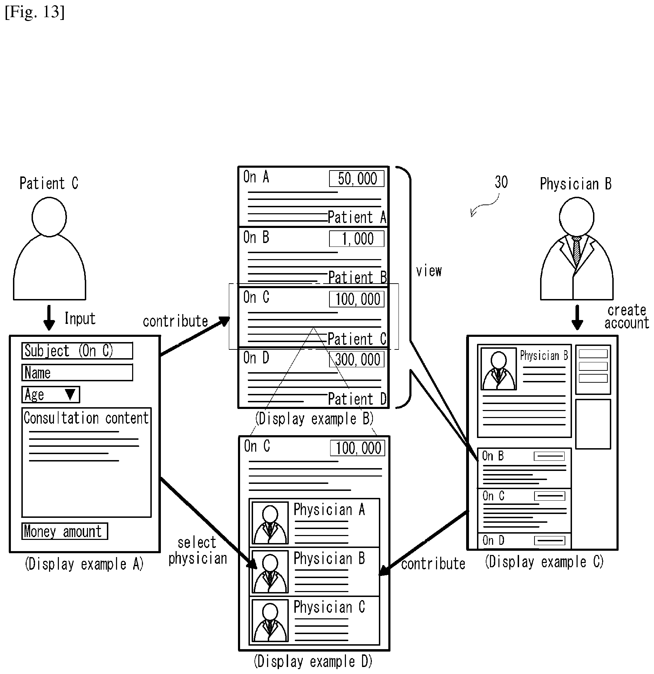

Moreover, when the patient and the physician are matched with each other in this embodiment, a physician to be matched is output to the patient (the target physician is displayed on the display 13 of the patient), but it may be vice versa, and a patient to be matched may be output to the physician (the patient is displayed on the display 13 of the target physician). Fig. 13 is a view conceptually illustrating a medical system 30 according to a variation 1. However, it is assumed that configuration without particular description is similar to that in the embodiment, and the description will be omitted, and the constituent elements similar to those in the embodiment are given the same reference numerals used in the embodiment as necessary. Description will be similarly omitted as appropriate in a variation 2 and a variation 3 which will be described later, and the same reference numerals used in the embodiment are given in the description.

As illustrated in this Fig. 13, first, when a patient (a patient C in Fig. 13) enters patient information relating to the patient (subject, name, age, consultation contents (name of a suffering disease, current symptom, previous treatments and the like) and a money amount in Fig. 13) through an input form (display example A) and transmits it, input contents of this input form are stored in the data recording unit 23 (not shown in Fig. 13) of the management server 20. When other patients (patient A, patient B, and Patient D and the like) also made inputs through the input form, the input contents from various patients are accumulated in the data recording unit 23. In Fig. 13, for example, the input contents "on A" by the patient A, "on B" by the patient B, "on C" by the patient C, and "on D" by the patient D and the like are accumulated (display example B).

On the other hand, when a physician create an account by registering various types of information relating to the physician (specialized field, treatment method and the like similar to the embodiment), a WEB page (display example C) displaying the various types of information is generated, and the input contents of the aforementioned patient can be viewed from this WEB page. The aforementioned input contents may be displayed on top of the WEB page, for example. The input contents displayed at this time preferably display only input contents with close relations with the physician and may display only the input contents relating to the specialized field of the physician on the basis of the input contents of the patient (name of a suffering disease, for example) and the physician's information (specialized field, for example).

As an example, first, the patient disease condition acquisition unit 22a (not shown in Fig. 13) specifies disease condition (disease name, for example) of the patient on the basis of the input contents input by the patient. If a specific disease name is entered through the input contents, the disease name may be specified, or if a vague symptom is entered, a DB storing a symptom and a disease name in association (not shown. See a symptom DB in Fig. 5 described later) may be referred to, and the disease name may be specified on the basis of the symptom. Then, the control unit 22 of the management server 20 specifies a physician specialized in the specified disease name by referring to the physician evaluation DB 23b (not shown in Fig. 13) and the like, and simplified pages of the input contents of the patient (only first several lines of a subject, a money amount, and consultation contents as in the display example B, for example) are listed and displayed on the WEB page of the specified physician (the display example C). The display example C illustrates an example in which the control unit 22 of the management server 20 determines that, since the input contents of "on A" do not match the specialty of the physician, they are not display targets and displays only "on B, "on C", and "on D". The physician can select a patient suitable for his/her treatment from them and transmit a message to the selected patient. By clicking the subject of the display example B, for example, a detailed page (display example D) of the clicked input contents is displayed, and a message can be written in a message column provided on this detailed page. On a page (display example D) of "on C", messages not only by the physician B but also the physician A and the physician C are written (That is, the physician A and the physician C also want to treat the patient C. This message column may be set so that those other than the patient cannot view it.

Then, the patient sees a message of each physician (display example D) to the input contents of himself/herself and can select the physician from whom the patient wants to receive treatment and exchange messages with the physician. At this time, it can be so configured that, by selecting a message column of the physician, for example, the patient can view the WEB page of the physician (display example C) so that the patient can check the specialized field, the evaluation and the like of the physician. As described above, the control unit 22 according to this variation 1 extracts the input contents ("on B", "on C", and "on D" in the display example B) relating to the medical treatment of the physician from the input contents of a plurality of patients (all in the display example B) and outputs the extracted input contents to the physician so as to match the patient and the physician and thus, the physician can select a patient suitable for a treatment, while the patient can select a physician by referring to the message from each physician and can select a physician more suitable for himself/herself on the basis of the physician information relating to the physician.

According to this variation 1, after the disease condition of the patient is specified on the basis of the input contents of the patient, the input contents are output to the physician determined to be appropriate on the basis of the disease condition and thus, the physician can contact the patient suitable for the specialty of the physician upon reception of the input contents, and the physician can select a patient appropriate for his/her own techniques easily.

### (Coordinator)

In this embodiment, a physician who gives treatment suitable for the patient is extracted, but this is not limiting, and a coordinator who introduces a physician suitable for the patient may be extracted. The "coordinator" introduces a physician to a patient and a so-called general practitioner or the like plays the role. Such coordinator requires capability of ascertaining seriousness of the disease of a patient at an initial stage of medical treatment and of introducing an appropriate special practitioner. A specific method of extracting such coordinator is arbitrary, but in the following, description will be made by assuming that the extraction is made on the basis of a coordinator DB storing evaluations relating to the coordinators.

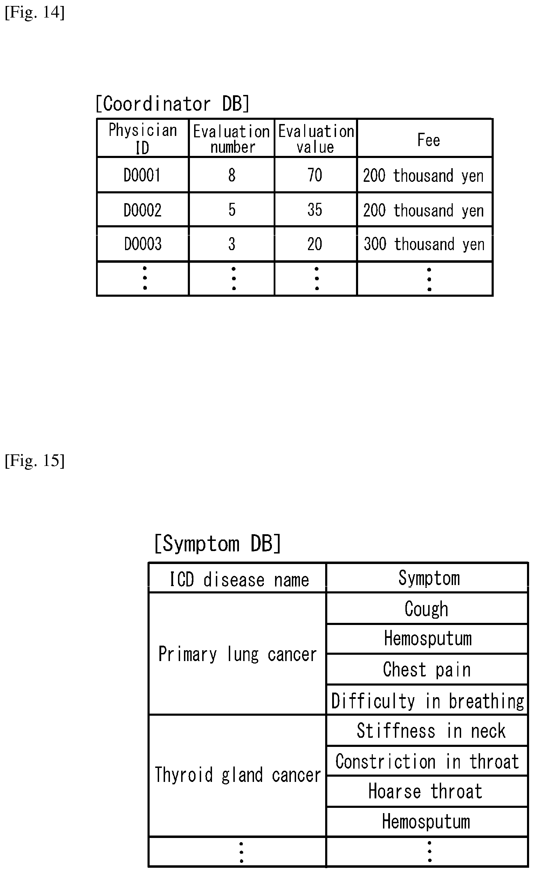

The coordinator DB is physician evaluation storage unit for storing physician evaluation information specifying evaluation specific to a physician and is coordinator evaluation storage unit for storing particularly coordinator information specifying evaluation of capability of the physician as a coordinator. Fig. 14 is a view illustrating a configuration example of the coordinator DB according to a variation 2. As illustrated in Fig. 14, the coordinator DB is configured by associating information corresponding to an item "physician ID", information corresponding to an item "evaluation number", information corresponding to an item "evaluation value", and information corresponding to an item "fee" with each other. Here, the information corresponding to the item "physician ID" is information for uniquely identifying a physician. The information corresponding to the item "evaluation number" is the number of persons who made evaluation of a work of the physician as a coordinator (hereinafter referred to as an evaluator), and numeral values such as "8", "5", "3" or the like are stored. The information corresponding to the item "evaluation value" expresses evaluation of the work of the physician as a coordinator in a numeral value and is a value calculated on the basis of the evaluation made by an evaluator for the work of the physician as a coordinator. A determination method of this evaluation value is arbitrary and three-stage evaluation may be made by a patient, for example, similarly to the physician evaluation processing in the embodiment. Moreover, the information corresponding to the item "fee" is information specifying a fee borne by a patient when receiving the work of each physician as a coordinator and information indicating a specific money amount such as "200 thousand yen" and "300 thousand yen".

Then, the control unit 15 of the terminal device 10 (not shown in Fig. 14) outputs an optimal coordinator for a patient on the basis of information stored in the coordinator DB. Since such output can be performed similarly to the aforementioned target physician output processing, an example will be briefly described in the following. First, since the coordinator plays a role of ascertaining the disease or the like as described above, it is assumed that a physician is preferably located in the neighborhood where the patient can easily consult the physician, and matching between the patient and the physician is made by considering the locations of the patient and the physician. Specifically, first, when the patient inputs the patient attribute information ("location", for example) in the terminal device 10, a physician having required time from the location of the patient within a predetermined time (1 hour, for example) is extracted. A specific method of such extraction is arbitrary, and the required time from the location of the aforementioned input patient attribute information to the location of a hospital to which the physician belongs stored as the information corresponding to the item "district" in the physician attribute DB 23c in Fig. 4 may be calculated by using a known route search application and it may be determined whether the required time is within the predetermined time similarly to the embodiment, for example. Then, from the extracted plurality of physicians (that is, the physicians located in the neighborhood of the patient), physicians having the evaluation values stored in correspondence with the item "evaluation value" in the coordinator DB at a reference value ("30", for example) or more are extracted and displayed on the display 13 of the terminal device 10(not shown in Fig. 14) in the order from those with the higher evaluation value. Then, the patient can select an appropriate physician in taking treatment for his/her disease from the physicians displayed as above. By combining the variation 2 and the embodiment, both the appropriate coordinator and the appropriate target physician for the patient may be output separately or a physician falling under both the appropriate coordinator and the target physician may be output.

### (ICD disease name)

In the target physician output processing in this embodiment, a list of the ICD disease names is displayed on the display 13 of the terminal device 10, and the patient selects the ICD disease name indicating the patient's disease from this list and inputs the ICD disease name, but the patient does not necessarily recognize his/her ICD disease name. Thus, the possible disease name (ICD disease name, for example) may be automatically extracted by the patient's inputting symptoms involved in his/her disease. A specific method of this specification is arbitrary, but it is assumed that the extraction is made on the basis of the symptom DB in the following description.

The symptom DB is symptom storage unit for storing disease name information specifying a disease name (hereinafter, ICD disease name) and symptom information specifying a symptom involved in the disease in association. Fig. 15 is a view illustrating a configuration example of the symptom DB according to a variation 3. As illustrated in Fig. 15, the symptom DB is configured by associating information corresponding to an item "ICD disease name" and information corresponding to an item "symptom" with each other. Here, the information corresponding to the item "ICD disease name" is ICD disease name specifying information specifying the ICD disease name, and "primary lung cancer" and "thyroid gland cancer", for example, are applicable. The information corresponding to the item "symptom" is a symptom involved in each disease, and "cough", "hemosputum", "chest pain", "difficulty in breathing" and the like are applicable. These types of information have been downloaded and stored in the symptom DB in advance via the internet or the like.

Then, in the target physician output processing, the control unit 15 of the terminal device 10 (not shown in Fig. 15) first determines whether an input of symptom information has been accepted. A specific method of this determination is arbitrary, and a list of the symptoms is displayed on the display 13 of the terminal device 10 (not shown in Fig. 15), for example, and it is determined that the input has been accepted when the patient selects the symptom applicable to himself/herself from this list. Then, by referring to the input symptom and the information of the aforementioned symptom DB, the ICD disease name of the patient is specified. When a plurality of symptoms are input, for example, a matching number (or a matching rate) between the plurality of symptoms and the item "symptom" of the symptom DB may be calculated, and the ICD disease name with the largest matching number may be specified as the ICD disease name of the patient.

According to the variation 3, since the disease name of the patient can be specified on the basis of the symptom of the patient, and the physician corresponding to the specified disease name and the patient can be matched with each other, even if the patient does not grasp the disease name or cannot determine by himself/herself whether the patient is sick or not, the patient and the physician can be matched with each other appropriately.

### (Note)

A medical system of note 1 comprises: patient disease condition acquisition unit for acquiring patient disease condition information specifying the disease condition of a patient; physician information storage unit for storing physician information relating to a physician; and matching unit for matching the patient and the physician with each other on the basis of the patient disease condition information acquired by the patient disease condition acquisition unit and the physician information stored in the physician information storage unit.

Moreover, a medical system in note 2 is the medical system described in note 1, in which: the physician information storage unit comprises physician evaluation storage unit for storing physician evaluation information specifying evaluation specific to the physician; the matching unit comprises physician extraction unit for extracting a target physician who is a physician suitable for medical treatment of the patient on the basis of the patient disease condition information acquired by the patient disease condition acquisition unit and the physician evaluation information stored in the physician evaluation storage unit; and the medical system comprises target physician information storage unit for storing information relating to the target physician extracted by the physician extraction unit and output unit for outputting information relating to the target physician stored in the target physician information storage unit.

Moreover, a medical system in note 3 is the medical system described in note 2, in which: the patient disease condition information acquired by the patient disease condition acquisition unit is an ICD disease name which is a disease name determined in accordance with the International Classification of Diseases; and the physician evaluation storage unit stores evaluation of the physician in relation to a disease corresponding to the ICD disease name as the physician evaluation information.

Moreover, a medical system in note 4 is the medical system described in note 2 or 3, in which: the physician evaluation storage unit stores evaluation of the physician in relation to a treatment method corresponding to disease condition of the patient as the physician evaluation information.

Moreover, a medical system in note 5 is the medical system described in any one of notes 2 to 4, comprising: patient attribute acquisition unit for acquiring patient attribute information specifying an attribute of the patient; physician attribute acquisition unit for acquiring physician attribute information specifying an attribute of the physician; and matching-degree calculation unit for calculating a matching degree indicating a degree for the physician to be suitable for treating the patient on the basis of the patient attribute information acquired by the patient attribute acquisition unit and the physician attribute information acquired by the physician attribute acquisition unit, and the physician extraction unit extracts the target physician on the basis of the matching degree calculated by the matching-degree calculation unit, the patient disease condition information acquired by the patient disease condition acquisition unit, and the physician evaluation information stored in the physician evaluation storage unit.

Moreover, a medical system in note 6 is the medical system described in note 5, in which the attribute of the patient includes the age, sex, current location or medical treatment fee of the patient.

Moreover, a medical system in note 7 is the medical system described in any one of notes 2 to 6, the evaluation specific to the physician is evaluation by physicians other than the physician concerned.

Moreover, a medical system in note 8 is the medical system described in any one of notes 1 to 7, comprising symptom storage unit for storing disease name information specifying a disease name and symptom information specifying a symptom involved in the disease in association with each other, and the patient disease condition acquisition unit acquires a symptom involved in the disease of the patient, and the matching unit specifies the disease name information stored in the symptom storage unit on the basis of the symptom acquired by the patient disease condition acquisition unit and the symptom information stored in the symptom storage unit and matches the patient and the physician with each other on the basis of the specified disease name information and the physician information stored in the physician information storage unit.

Moreover, a medical system in note 9 is the medical system described in any one of notes 1 to 8, in which: a plurality of patients makes inputs relating to symptoms of their own disease conditions by input unit; the patient disease condition acquisition unit specifies the disease condition of each of the patients on the basis of the input contents input by the patients; and the matching unit matches each of the patients and the physician with each other by extracting input contents relating to the medical treatment of the physician from the input contents of the plurality of patients on the basis of the physician information relating to the physician, and outputting the extracted input contents to the physician.

Moreover, a medical program in note 10 causes a computer to function as: patient disease condition acquisition unit for acquiring patient disease condition information specifying disease condition of a patient; physician information storage unit for storing physician information relating to a physician; and matching unit for matching the patient and the physician with each other on the basis of the patient disease condition information acquired by the patient disease condition acquisition unit and the physician information stored in the physician information storage unit.

### (Advantageous Effects of notes)

According to the medical system described in note 1 or the medical program described in note 10, since the patient and the physician are matched with each other on the basis of the disease condition information and the physician information, the matching of the both can be made on the basis of the disease condition of the patient and the physician's information, the patient can select a physician appropriate for his/her own disease and the physician can select an appropriate patient in accordance with his/her own techniques.

According to the medical system described in note 2, since the information relating to the target physician is output on the basis of the patient disease condition information and the physician evaluation information, a physician determined to be objectively excellent can be output with priority in selecting a target physician, and the patient can select a physician more appropriate for his/her own disease.

According to the medical system described in note 3, since the evaluation of the physician in relation to a disease corresponding to the ICD disease name is stored as the physician evaluation information, the evaluation of the physician relating to the internationally prescribed reference can be stored, and a single integrated evaluation can be stored even for a disease having a plurality of disease names.

According to the medical system described in note 4, since the evaluation of the physician in relation to a treatment method corresponding to the disease condition of the patient is stored as the physician evaluation information, even if a plurality of different diseases can be treated by a single treatment method or the like, the evaluation of a physician can be appropriately stored.

According to the medical system described in note 5, the matching degree indicating a degree for the physician to be suitable for treating the patient is calculated on the basis of the patient attribute information and the physician attribute information, and the target physician is extracted by considering this matching degree and thus, the target physician can be extracted on the basis of the information other than the patient's disease condition or the evaluation of the physician and the information specific to the patient and the physician, and the patient can select a physician more appropriate for treatment for the patient's own disease.

According to the medical system described in note 6, since the attribute of the patient includes age, sex, a current location or a medical treatment cost of the patient, the target physician can be extracted on the basis of the specific information such as the age, sex, current location or a medical treatment cost of the patient, and the patient can select a physician more appropriate for treatment for the patient's own disease.

According to the medical system described in note 7, since the evaluation of the physician is determined on the basis of evaluation from another physician who has specialized knowledge, more appropriate evaluation can be stored.

According to the medical system described in note 8, since the disease name of the patient can be specified on the basis of the symptom of the patient, and the physician corresponding to the specified disease name and the patient can be matched with each other, even if the patient does not grasp the disease name or cannot determine by himself/herself whether the patient is sick or not, the patient and the physician can be matched with each other appropriately.

According to the medical system described in note 9, after the disease condition of the patient is specified on the basis of the input contents of the patient, the input contents are output to the physician determined to be appropriate on the basis of the disease condition and thus, the physician can contact the patient suitable for the specialty of the physician upon reception of the input contents, and the physician can select a patient appropriate for his/her own techniques easily.

### [Description of Reference Numerals and Signs]

- 1: medical system
- 2: network
- 10: terminal device
- 11: communication unit
- 12: operation unit
- 13: display
- 14: speaker
- 15: control unit
- 16: data recording unit
- 20: management server
- 21: communication unit
- 22: control unit
- 22a: patient disease condition acquisition unit
- 22b: patient attribute acquisition unit
- 22c: physician attribute acquisition unit
- 22d: matching degree calculation unit
- 22e: physician extraction unit
- 23: data recording unit
- 23a: ICD disease name DB
- 23b: physician evaluation DB
- 23c: physician attribute DB
- 23d: target physician DB
- 30: medical system

## Claims

1. A medical system comprising:
patient disease condition acquisition unit for acquiring patient disease condition information specifying disease condition of a patient;
physician information storage unit for storing physician information relating to a physician; and
matching unit for matching the patient and the physician with each other on the basis of the patient disease condition information acquired by the patient disease condition acquisition unit and the physician information stored in the physician information storage unit.

2. The medical system according to claim 1, wherein
the physician information storage unit comprises physician evaluation storage unit for storing physician evaluation information specifying evaluation specific to the physician;
the matching unit comprises physician extraction unit for extracting a target physician who is a physician suitable for medical treatment of the patient on the basis of the patient disease condition information acquired by the patient disease condition acquisition unit and the physician evaluation information stored in the physician evaluation storage unit; and
the medical system comprises:
target physician information storage unit for storing information relating to the target physician extracted by the physician extraction unit; and
output unit for outputting information relating to the target physician stored in the target physician information storage unit.

3. The medical system according to claim 2, wherein
the patient disease condition information acquired by the patient disease condition acquisition unit is an ICD disease name, which is a disease name determined in accordance with the International Classification of Diseases; and
the physician evaluation storage unit stores evaluation of the physician in relation to a disease corresponding to the ICD disease name as the physician evaluation information.

4. The medical system according to claim 2 or 3, wherein
the physician evaluation storage unit stores evaluation of the physician in relation to a treatment method corresponding to the disease condition of the patient as the physician evaluation information.

5. The medical system according to any one of claims 2 to 4, further comprising:
patient attribute acquisition unit for acquiring patient attribute information specifying an attribute of the patient;
physician attribute acquisition unit for acquiring physician attribute information specifying an attribute of the physician; and
matching degree calculation unit for calculating a matching degree indicating a degree for the physician to be suitable for treating the patient on the basis of the patient attribute information acquired by the patient attribute acquisition unit and the physician attribute information acquired by the physician attribute acquisition unit, wherein
the physician extraction unit extracts the target physician on the basis of the matching degree calculated by the matching degree calculation unit, the patient disease condition information acquired by the patient disease condition acquisition unit, and the physician evaluation information stored in the physician evaluation storage unit.

6. The medical system according to claim 5, wherein
the attribute of the patient includes age, sex, a current location or a medical treatment fee of the patient.

7. The medical system according to any one of claims 2 to 6, wherein
the evaluation specific to the physician is evaluation by physicians other than the physician concerned.

8. The medical system according to any one of claims 1 to 7, further comprising: symptom storage unit for storing, in association with each other, disease name information specifying a disease name and symptom information specifying a symptom involved in the disease, wherein
the patient disease condition acquisition unit acquires a symptom involved in the disease of the patient; and
the matching unit specifies the disease name information stored in the symptom storage unit on the basis of the symptom acquired by the patient disease condition acquisition unit and the symptom information stored in the symptom storage unit, and matches the patient and the physician with each other on the basis of the specified disease name information and the physician information stored in the physician information storage unit.

9. The medical system according to any one of claims 1 to 8, wherein
a plurality of the patients makes inputs relating to their own disease conditions by input unit,
the patient disease condition acquisition unit specifies the disease condition of each of the patients on the basis of input contents input by the patients; and
the matching unit matches each of the patients and the physician with each other by: extracting input contents relating to medical treatment of the physician from the input contents of the plurality of patients on the basis of the physician information relating to the physician; and outputting the extracted input contents to the physician.

10. A medical program which causes a computer to function as:
patient disease condition acquisition unit for acquiring patient disease condition information specifying disease condition of a patient;
physician information storage unit for storing physician information relating to a physician; and
matching unit for matching the patient and the physician with each other on the basis of the patient disease condition information acquired by the patient disease condition acquisition unit and the physician information stored in the physician information storage unit.
